# EUROPEAN PATENT APPLICATION

(11) **EP 4 279 063 A2**
(43) Date of publication of application: **22.11.2023**
(21) Application number: 23193774.9
(22) Date of filing: 22.01.2019
(51) Int. Cl.: A61K 9/00

(54) **OPHTHALMIC PHARMACEUTICAL COMPOSITION, PREPARATION METHOD THEREFOR AND APPLICATION THEREOF**

(30) Priority: 23.01.2018 CN 201810063904
(62) Divisional of application: 19743390.7
(71) Applicant: Seinda Pharmaceutical Guangzhou Corporation, Guangzhou, Guangdong (CN)
(72) Inventor: LIU, Zuguo, Xiamen (CN); ZHAO, Yufen, Xiamen (CN); HUANG, Caihong, Xiamen (CN); WU, Yang, Xiamen (CN); TANG, Guo, Xiamen (CN); LIU, Yan, Xiamen (CN); XU, Pengxiang, Xiamen (CN)
(74) Representative: Fish & Richardson P.C.

(57) **Abstract**

The invention relates to an ophthalmic pharmaceutical composition and its preparation methods and applications, wherein the pharmaceutical composition comprises of L-Alanyl-L-Glutamine suspended or dissolved in an isoosmotic solution suitable for human eyes, and has the advantages causing minimal irritation, with high stability and a good safety profile, and which can be used for relieving dry eye disease (DED) symptoms and/or improving and/or treating DED.

## Description

### TECHNICAL FIELD

The present invention relates to the technical field of pharmaceutical preparations, more particularly, an ophthalmic pharmaceutical composition and methods of its preparation thereof, and the applications in ophthalmic preparations for ameliorating the symptoms of dry eye disease (DED) and/or improving DED and/or treating DED.

### BACKGROUND

DED refers to a class of diseases caused by abnormal tear film or ocular surface homeostasis, which result in tear film instability, eye discomfort and visual impairment. It has the potential to cause permanent damage to the ocular surface. DED usually manifests as dryness, pain, burning and itchiness in the eye as well as foreign body sensation, blurred vision, red eyes, photophobia, tearing, etc. Long-term eye discomfort can seriously affect the patient's quality of life. Severe DED disease may also lead to keratitis, corneal neovascularization, corneal ulcers, and may even threaten the patient's vision and lead to eventual blindness. Currently, DED is the most common ocular surface disease worldwide. Epidemiological investigation shows that the incidence of DED ranges from 5.5% to 33.7% in the world and 21% to 30% in China. It is estimated that there are more than 300 million DED patients in China. In recent years, with the aggravation of air pollution and the increasing use of video terminals and digital screens, the prevalence of DED is increasing rapidly and developing at an increasingly younger age. DED may cause difficulties for patients in their daily work and activities, such as reading, using computers, watching TV and driving. DED thus has significant negative impact on people's work efficiency and quality of life.

There are many factors affecting the occurrence and development of DED. Factors that induce DED include ocular surface inflammation, drug abuse, long-term corneal contact lens-wear, menopause, long-term computer use and other immune factors. Studies have shown that the main pathological manifestations of DED are a series of damages to the ocular surface epithelium induced by dry environmental stress after tear film homeostasis is reduced, including impairment of corneal epithelial barrier function, decrease of conjunctival goblet cell density, ocular surface squamous metaplasia and ocular surface inflammation. Currently, the main treatments for DED include artificial tears, corticosteroids and immunosuppressants such as cyclosporine A (CsA). Artificial tears, however, are only a substitute for natural tears and have no therapeutic effect. Long-term use of corticosteroids and CsA have certain toxic side-effects on the ocular surface. So far, there is no particularly efficacious drug for the treatment of DED in clinical practice. Therefore, there is an urgent need to develop a drug with good efficacy, long-term use and no obvious local and systemic side effects for the treatment of DED.

### SUMMARY

This invention provides an ophthalmic pharmaceutical composition, its methods of preparation, and its applications in preparing ophthalmic preparations for ameliorating the symptoms of DED and/or the improvement and/or the treatment of DED. The ophthalmic pharmaceutical composition of the invention has the advantages of causing minimal irritation, with high stability and a good safety profile.

This invention is achieved by the following technical scheme:
Firstly, the invention provides an ophthalmic pharmaceutical composition, which comprises of L-Alanyl-L-Glutamine suspended or dissolved in an acceptable isoosmotic ophthalmic solution.

Preferably, the concentration of L-Alanyl-L-Glutamine is in the range of 0.1-10% (w/v), preferably 1-10% (w/v), more preferably 1-5% (w/v), and most preferably 1% (w/v).

Preferably, the isoosmotic solution is prepared with an osmotic agent;
Preferably, the osmotic agent is selected from one or more of the following: sodium chloride, potassium chloride, boric acid, borax, sodium sulfate, potassium sulfate, sodium nitrate, potassium nitrate, sodium acetate, mannitol, glycerin, propylene glycol, 2-(4-octylphenylethyl)-2-amino-propylene glycol hydrochloride and glucose;
Preferably, the osmotic agent is selected from either or both sodium chloride and potassium chloride;
Preferably, the osmotic agent is sodium chloride;
Preferably, the concentration of the osmotic agent is 0.01-3% (w/v), preferably 0.1-1% (w/v), more preferably 0.4-0.8% (w/v), and most preferably 0.5%(w/v).

Preferably, the pharmaceutical composition optionally contains a bacteriostatic or antimicrobial agent;

Preferably, the bacteriostatic or antimicrobial agent is ethyl hydroxybenzoate;
Preferably, the concentration of the bacteriostatic or antimicrobial agent is 0.003-0.5% (w/v), preferably 0.01-0.05% (w/v), more preferably 0.02-0.035% (w/v), most preferably 0.03%(w/v).

Preferably, the pharmaceutical composition optionally includes a viscosity modifying agent;
Preferably, the viscosity modifying agent is selected from one or more of the following: sodium hyaluronate, sodium carboxymethyl cellulose, methyl cellulose, polyethylene glycol, polyvinyl alcohol and povidone;
Preferably, the viscosity modifying agent is selected from either or both sodium hyaluronate and sodium carboxymethyl cellulose;
Preferably, the viscosity modifying agent is sodium hyaluronate.

Preferably, the concentration of the viscosity modifying agent is 0.01-0.5% (w/v), preferably 0.05-0.2% (w/v), more preferably 0.1-0.15% (w/v), most preferably 0.1%(w/v).
Preferably, the pharmaceutical composition also includes one or more pH regulators selected from the following: sodium dihydrogen phosphate, disodium hydrogen phosphate, potassium dihydrogen phosphate, dipotassium hydrogen phosphate, boric acid, borax, acetic acid, sodium acetate, citric acid, sodium citrate, tartaric acid, sodium tartrate, sodium carbonate, potassium carbonate, sodium bicarbonate, potassium bicarbonate, sodium hydroxide, potassium hydroxide, hydrochloric acid and phosphoric acid. The pH regulator adjusts the pH value to 5.0-9.0, preferably to 6.0-8.0, more preferably to 6.5-7.5, and most preferably to 7.0;
Preferably, the pH adjusting agent is selected from one or more of the following: sodium carbonate, sodium bicarbonate and sodium hydroxide.

Preferably, the pH adjusting agent is sodium hydroxide;
Preferably, the concentration of sodium hydroxide is 0.25 mol/L.

Secondly, the invention also provides a method for preparing the pharmaceutical composition. The method comprises of the following steps: suspending or dissolving the propionic dipeptide in an isoosmotic solution; adjusting the pH value to 5.0-9.0, preferably to 6.0-8.0, more preferably to 6.5-7.5, and most preferably to 7.0; and filtering to sterilize the solution with a microporous filter membrane;
Preferably, the method comprises of the following steps: stirring and dissolving the osmotic agent with water for injection, preferably at 80-90°C, most preferably at 85°C; adding L-Alanyl-L-Glutamine and stirring to dissolve; adding water for injection; adjusting the pH value to 5.0-9.0 with pH adjusting agent, preferably to 6.0-8.0, more preferably to 6.5-7.5, most preferably to 7.0; heating to sterilize at 100°C; filtering to sterilize with a microporous filter membrane; and filling into sterilized eye drops bottles in a aseptic manufacturing environment;
Preferably, the method comprises of the following steps: stirring and dissolving osmotic agents and bacteriostatic or antimicrobial agents with water for injection, preferably at 80-90°C, most preferably at 85°C; after dissolving, optionally adding a viscosity modifying agent while stirring and continuing to stir until dissolved; adding L-Alanyl-L-Glutamine and stirring to dissolve; adding water for injection; adjusting the final pH value to 5.0-9.0 with pH adjusting agent, preferably to 6.0-8.0, more preferably to 6.5-7.5, most preferably to 7.0; heating to sterilize at 100°C; filtering to sterilize with a microporous filter membrane; and filling into sterilized eye drops bottles under aseptic manufacturing environment;
Thirdly, the present invention also provides applications of the above pharmaceutical composition in preparing ophthalmic preparations for ameliorating the symptoms of DED and/or improving DED and/or treating DED.

### DRAWINGS

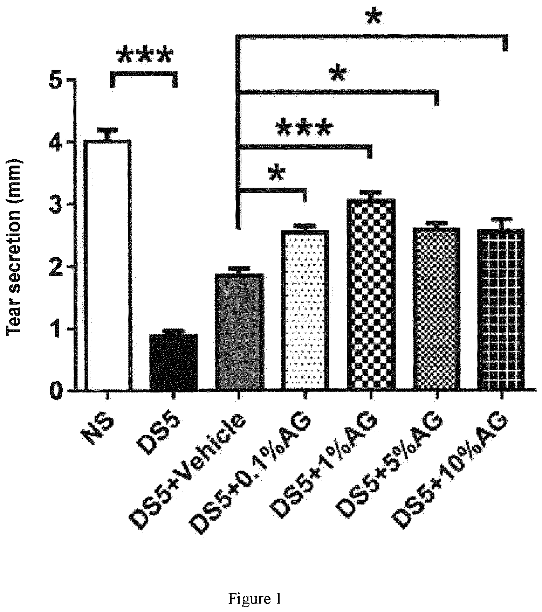

Figure 1 shows the statistical analysis of tear secretion in mice. NS represented normal group, DSS represented DED group, DSS+Vehicle represented solvent control group, and DSS+AG represented treatment group; *P<0.05; ** P<0.01; ***P<0.001;

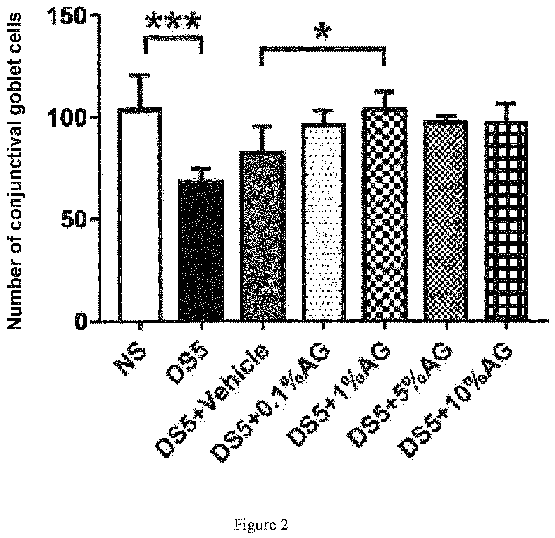

Figure 2 shows the statistics of the number of conjunctival goblet cells in mice. NS represented the normal group, DSS represented the DED group, DSS+Vehicle represented the solvent control DED group, and DSS+AG represented the DED treatment group treated with ophthalmic preparation; ^{∗}P<0.05 ; ^{∗∗}P<0.01; ^{∗∗∗}P<0.001;

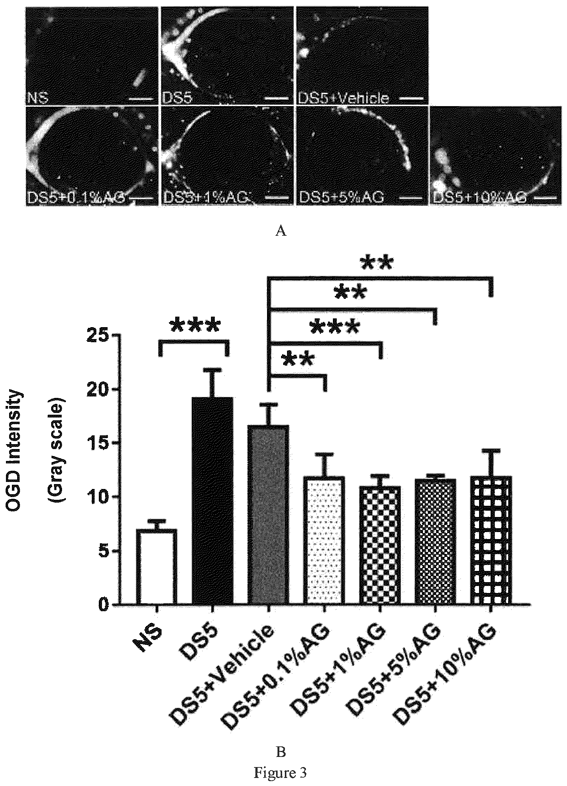

Figure 3 shows the corneal epithelial defect in mice. Figure 3A was a typical image of mouse corneal OGD staining. Figure 3B was the statistics of the fluorescence intensity of mouse corneal OGD staining. NS represented normal group, DSS represented DED group, DSS+ Vehicle represented the solvent control group, and DS5+AG represented the DED treatment group; *P<0.05; **P<0.01; ***P<0.001.

### DETAILED DESCRIPTION

The technical solutions of this invention will be specified in the form of embodiments below, but the following examples or experimental examples do not limit the scope of the present invention.

Unless otherwise specified, the embodiments in the following examples are conventional methods, and the raw materials and reagent materials used in the following examples are all commercially available products.

### Preparation example 1

1. Pharmaceutical composition

| | |
|---|---|
| Sodium hyaluronate | 1.0 g |
| Ethyl hydroxybenzoate | 0.3 g |
| Sodium chloride | 5.0 g |
| L-Alanyl-L-Glutamine | 10.0 g |
| Water for injection | Add water until total volume reaches 1000mL |
| pH | Adjust pH to 7.0 with 0.25 mol/L sodium hydroxide |

2. Preparation method

Weigh sodium chloride and ethyl hydroxybenzoate, then add 800 mL (85°C) of water and stir to dissolve. After dissolving, add sodium hyaluronate while stirring and continue to stir until dissolved, then add L-Alanyl-L-Glutamine and stir to dissolve. Add water until reaching a total volume of 1000 mL, then add sodium hydroxide to adjust the pH value to 7.0. Boil and sterilize at 100 °C for 30 minutes, then filter with 0.22 µm microporous filter membrane, and aseptically dispense into sterilized eye dropper bottles, 5 ml/bottle.

### Preparation example 2

1. Pharmaceutical composition

| | |
|---|---|
| Sodium hyaluronate | 1.2 g |
| Ethyl hydroxybenzoate | 0.3 g |
| Sodium chloride | 5.0 g |
| L-Alanyl-L-Glutamine | 0.2 g |
| Water for injection | Add water until total volume reaches 1000mL |
| pH | Adjust pH to 7.0 with 0.25 mol/L sodium hydroxide |

2. The preparation method is the same as Preparation example 1

### Preparation example 3

1. Pharmaceutical composition

| | |
|---|---|
| Sodium hyaluronate | 1.5 g |
| Ethyl hydroxybenzoate | 0.35 g |
| Sodium chloride | 6.0 g |
| L-Alanyl-L-Glutamine | 50.0 g |
| Water for injection | Add water until total volume reaches 1000mL |
| pH | Adjust pH to 7.5 with 0.25 mol/L sodium hydroxide |

2. The preparation method is the same as Preparation example 1.

### Preparation example 4

1. Pharmaceutical composition

| | |
|---|---|
| Sodium hyaluronate | 1.0 g |
| Ethyl hydroxybenzoate | 0.2 g |
| Sodium chloride | 2.0 g |
| L-Alanyl-L-Glutamine | 10.0 g |
| Water for injection | Add water until total volume reaches 1000mL |
| pH | Adjust pH to 6.5 with 0.25 mol/L sodium hydroxide |

2. The preparation method is the same as Preparation example 1.

### Preparation example 5

1. Pharmaceutical composition

| | |
|---|---|
| Sodium hyaluronate | 2.0 g |
| Ethyl hydroxybenzoate | 0.5 g |
| Sodium chloride | 10.0 g |
| L-Alanyl-L-Glutamine | 100.0 g |
| Water for injection | Add water until total volume reaches 1000mL |
| pH | Adjust pH to 8.0 with 0.25 mol/L sodium hydroxide |

2. The preparation method is the same as Preparation example 1.

### Preparation example 6

1. Pharmaceutical composition

| | |
|---|---|
| Sodium hyaluronate | 0.5 g |
| Ethyl hydroxybenzoate | 0.1 g |
| Sodium chloride | 1.0 g |
| L-Alanyl-L-Glutamine | 10.0 g |
| Water for injection | Add water until total volume reaches 1000mL |
| pH | Adjust pH to 6.0 with 0.25 mol/L sodium hydroxide |

2. The preparation method is the same as Preparation example 1.

### Preparation example 7

1. Pharmaceutical composition

| | |
|---|---|
| Sodium hyaluronate | 5.0 g |
| Ethyl hydroxybenzoate | 5.0 g |
| Sodium chloride | 30.0 g |
| L-Alanyl-L-Glutamine | 100.0 g |
| Water for injection | Add water until total volume reaches 1000mL |
| pH | Adjust pH to 9.0 with 0.25 mol/L sodium hydroxide |

2. The preparation method is the same as Preparation example 1.

### Preparation Example 8

1. Pharmaceutical composition

| | |
|---|---|
| Sodium hyaluronate | 0.1 g |
| Ethyl hydroxybenzoate | 0.03 g |
| Sodium chloride | 0.1 g |
| L-Alanyl-L-Glutamine | 1.0 g |
| Water for injection | Add water until total volume reaches 1000mL |
| pH | Adjust pH to 5.0 with 0.25 mol/L sodium hydroxide |

2. The preparation method is the same as Preparation example 1.

### Preparation Example 9

1. Pharmaceutical composition

| | |
|---|---|
| Sodium hyaluronate | 1.0 g |
| Ethyl hydroxybenzoate | 0.3 g |
| Sodium chloride | 5.0 g |
| L-Alanyl-L-Glutamine | 1.0 g |
| Water for injection | Add water until total volume reaches 1000mL |
| pH | Adjust pH to 7.0 with 0.25 mol/L sodium hydroxide |

2. The preparation method is the same as Preparation example 1.

### Preparation Example 10

1. Pharmaceutical composition

| | |
|---|---|
| Sodium hyaluronate | 1.0 g |
| Ethyl hydroxybenzoate | 0.3 g |
| Sodium chloride | 5.0 g |
| L-Alanyl-L-Glutamine | 50.0 g |
| Water for injection | Add water until total volume reaches 1000mL |
| pH | Adjust pH to 7.0 with 0.25 mol/L sodium hydroxide |

2. The preparation method is the same as Preparation example 1.

### Preparation Example 11

1. Pharmaceutical composition

| | |
|---|---|
| Sodium hyaluronate | 1.0 g |
| Ethyl hydroxybenzoate | 0.3 g |
| Sodium chloride | 5.0 g |
| L-Alanyl-L-Glutamine | 100.0 g |
| Water for injection | Add water until total volume reaches 1000mL |
| pH | Adjust pH to 7.0 with 0.25 mol/L sodium hydroxide |

2. The preparation method is the same as Preparation example 1.

### Preparation Example 12

1. Pharmaceutical composition

| | |
|---|---|
| Sodium carboxymethyl cellulose | 1.0 g |
| Benzalkonium chloride | 0.3 g |
| Potassium chloride | 5.0 g |
| L-Alanyl-L-Glutamine | 10.0 g |
| Water for injection | Add water until total volume reaches 1000mL |
| pH | Adjust pH to 7.0 with 0.25 mol/L sodium hydroxide |

2. Method

Weigh potassium chloride and benzalkonium chloride, then add 800 mL of water for injection (85°C) and stir to dissolve. After dissolving, add sodium carboxymethylcellulose while stirring, and continue to stir until dissolved. Add L-Alanyl-L-Glutamine, and stir to dissolve, then add water until reaching a total volume of 1000 mL. Add sodium hydroxide to adjust the pH to 7.0, and heat to sterilize at 100 °C for 30 minutes, then filter with 0.22 µm microporous filter membrane, and aseptically dispense into sterilized eye dropper bottles, 5 ml/bottle.

### Preparation Example 13

1. Pharmaceutical composition

| | |
|---|---|
| Sodium carboxymethyl cellulose | 1.0 g |
| Benzalkonium bromide | 0.3 g |
| Potassium chloride | 5.0 g |
| L-Alanyl-L-Glutamine | 10.0 g |
| Water for injection | Add water until total volume reaches 1000mL |
| pH | Adjust pH to 7.0 with 0.25 mol/L sodium hydroxide |

2. Method

Weigh potassium chloride and benzalkonium bromide, then add 800 mL of water (85°C) and stir to dissolve. After dissolving, add sodium carboxymethylcellulose while stirring and continue to stir until dissolved. Add L-Alanyl-L-Glutamine, and stir to dissolve, then add water until reaching a total volume of 1000 mL. Add sodium hydroxide to adjust pH to 7.0, and boil to sterilize at 100 °C for 30 minutes. Filter with 0.22 µm microporous filter membrane, and aseptically dispense into sterilized eye dropper bottles, 5 ml/bottle.

### Preparation Example 14

1. Pharmaceutical composition

| | |
|---|---|
| Ethyl hydroxybenzoate | 0.3 g |
| Sodium chloride | 5.0 g |
| L-Alanyl-L-Glutamine | 10.0 g |
| Water for injection | Add water until total volume reaches 1000mL |
| pH | Adjust pH to 7.0 with 0.25 mol/L sodium hydroxide |

2. Method

Weigh sodium chloride and Ethyl hydroxybenzoate, then add 800 mL of water for injection (85°C) and stir to dissolve. After dissolving, add L-Alanyl-L-Glutamine and stir to dissolve, then filter with 0.22 µm microporous filter membrane, and aseptically dispense into sterilized eye dropper bottles, 5 ml/bottle.

### Preparation Example 15

1. Pharmaceutical composition

| | |
|---|---|
| Sodium hyaluronate | 1.0 g |
| Sodium chloride | 5.0 g |
| L-Alanyl-L-Glutamine | 10.0 g |
| Water for injection | Add water until total volume reaches 1000mL |
| pH | Adjust pH to 7.0 with 0.25 mol/L sodium hydroxide |

2. Method

Weigh sodium chloride, then add 800 mL of water (85°C) and stir to dissolve. After dissolving, add sodium hyaluronate while stirring and continue to stir until dissolved, then add L-Alanyl-L-Glutamine and stir to dissolve. Adjust the pH to 7.0, heat to sterilize at 100 °C for 30 min, then filter with 0.22 µm microporous filter membrane, and aseptically dispense into sterilized eye dropper bottles, Sml/bottle.

### Preparation Example 16

1. Pharmaceutical composition

| | |
|---|---|
| Sodium chloride | 5.0 g |
| L-Alanyl-L-Glutamine | 10.0 g |
| Water for injection | Add water until total volume reaches 1000mL |
| pH | Adjust pH to 7.0 with 0.25 mol/L sodium hydroxide |

2. Method

Weigh sodium chloride, then add 800 mL of water for injection (85°C) and stir to dissolve. After dissolving, add L-Alanyl-L-Glutamine and stir to dissolve, then filter with 0.22 µm microporous filter membrane, and aseptically dispense into sterilized eye dropper bottles, 5 ml/bottle.

### Experimental example

### Animal experiment methods:

### (1) Establishment of DED model in mice and its grouping treatment

Healthy female C57/BL mice aged 10-12 weeks were randomly divided into 4 groups: Normal group (NS), DED group (DSS), Dry eye vehicle control group (DS5+Vehicle) and Dry eye treatment group (DSS +AG).

**Establishment of mouse DED model:** mice were kept in a dry environment (relative humidity: <40%, temperature: 21-23°C) and subcutaneous injection of scopolamine hydrobromide (0.5 mg/0.2 ml, 200 µl each, 4 times a day for 5 consecutive days), DED mice were induced successfully.

**Mice in the Normal group (NS):** The NS group included normal mice. This group of mice were not treated with eye drops and were kept in a standard environment at a temperature of 21-23°C and a relative humidity of 50-60%.

**Mice in the DED group (DS5):** The DED mice in the DED group did not receive eye drop treatment, they were kept in an environment with a temperature of 21-23°C and a relative humidity of less than 40% and received subcutaneous injection of scopolamine hydrobromide (0.5 mg /0.2 ml, 200 µl each time, 4 times a day for 5 consecutive days).

**Mice in the Dry eye vehicle control group (solvent only control) group (DS5 + Vehicle):** the eyes of DED mice in this group received the Vehicle Control eye drop (1 drop each time, 4 times a day, with an interval of 4 hours each time for 5 days). The mice were kept in a dry environment (relative humidity: <40%, temperature: 21-23°C).

**Mice in the Dry eye treatment group (DS5+AG):** The eyes of DED mice were treated with the ophthalmic formulations prepared in Preparation examples 1-16 (1 drop each time, 4 times a day, with an interval of 4 hours each time, and continued treatment for 5 days). The mice were kept in a dry environment (relative humidity: <40%, temperature: 21-23°C).

The handling of animals during the experiment was in conformance with the "Guiding Opinions on Treating Experimental Animals" issued by the Ministry of Science and Technology.

### (2) Determination of related indicators

After the treatment of the mice in each group, the mice were examined. Each examination was performed by the same person, and the time, place, lighting, and temperature were the same for each examination.

The tear secretion, corneal Oregongreen-dextran (OGD) staining and the number of conjunctival goblet cells were analyzed with the mice in each group.

### ① Phenol red cotton thread test to detect the amount of tear secretion

The amount of tear secretion was detected by phenolamine cotton (Zone-Quick; Lacrimedics, Eastsound, WA). Under the slit lamp, the phenolic cotton thread was placed in the inferior conjunctival fornix of the lateral canthus of mice with ophthalmic forceps. 15 seconds later, the length of phenolic cotton thread staining was measured with a millimeter ruler and recorded. The results are shown in Table 1.

**Table 1 Tear secretion (mm)**

| Preparation example | | Normal group (NS) | DED group (DSS) | Solvent control group (DS5+Vehicle) | | Treatment group (DS5+AG) | |
|---|---|---|---|---|---|---|---|
| | 1 | 4 | 0.86 | | 1.85 | | 3.04 |
| | 2 | | | | 1.71 | | 2.66 |
| | 3 | | | | 1.75 | | 2.57 |
| | 4 | | | | 1.73 | | 2.90 |
| | 5 | | | | 1.58 | | 2.45 |
| | 6 | | | | 1.64 | | 2.80 |
| | 7 | | | | 1.54 | | 2.40 |
| | 8 | | | | 1.55 | | 2.35 |
| | 9 | | | | 1.85 | | 2.53 |
| | 10 | | | | 1.85 | | 2.58 |
| | 11 | | | | 1.85 | | 2.54 |
| | 12 | | | | 1.53 | | 2.70 |
| | 13 | | | | 1.52 | | 2.68 |
| | 14 | | | | 1.45 | | 2.40 |
| | 15 | | | | 1.76 | | 2.83 |
| | 16 | | | | 1.47 | | 2.33 |

### (2) OGD staining to detect corneal epithelial barrier function:

0.5 µl of OGD (50 mg/ml, 70,000 molecular weight; Invitrogen) was applied to the lower conjunctival sac of mice, and the mice were sacrificed and the conjunctiva was rinsed with 1 mL of saline, and then under an *in vivo* fluorescence microscope (AZ100, Nikon) the fluorescence staining of corneal epithelium were analyzed and photographed. The fluorescence intensity of corneal staining was measured and recorded using NIS-element software. The results are shown in Table 2.

**Table 2 Staining fluorescence intensity (a.u.)**

| Preparation example | | Normal group (NS) | DED group (DSS) | Solvent control group (DS5+Vehicle) | | Therapy group (DS5+AG) | |
|---|---|---|---|---|---|---|---|
| | 1 | 6.87 | 19.09 | | 16.49 | | 10.76 |
| | 2 | | | | 16.58 | | 11.23 |
| | 3 | | | | 16.73 | | 11.53 |
| | 4 | | | | 16.64 | | 10.88 |
| | 5 | | | | 16.89 | | 11.81 |
| | 6 | | | | 16.90 | | 11.05 |
| | 7 | | | | 17.11 | | 11.93 |
| | 8 | | | | 17.24 | | 12.03 |
| | 9 | | | | 16.49 | | 11.70 |
| | 10 | | | | 16.49 | | 11.40 |
| | 11 | | | | 16.49 | | 11.74 |
| | 12 | | | | 17.45 | | 11.20 |
| | 13 | | | | 17.50 | | 11.17 |
| | 14 | | | | 17.62 | | 12.23 |
| | 15 | | | | 17.20 | | 11.15 |
| | 16 | | | | 17.70 | | 12.34 |

### ③. Measurement of the number of goblet cells:

Eye tissue specimens were fixed with 10% formalin and embedded in paraffin and sectioned. The sections were stained with periodic acid-Schiff (PAS) reagent. Nikon Nikon eclipse 50i was used to collect images and the number of conjunctival goblet cells were counted. The results are shown in Table 3.

**Table 3 Number of goblet cells in conjunctiva**

| Preparation example | | Normal group (NS) | DED group (DS5) | Solvent control group (DS5+Vehicle) | | Treatment group (DS5+AG) | |
|---|---|---|---|---|---|---|---|
| | 1 | 103.6 | 68.19 | | 82.48 | | 103.5 |
| | 2 | | | | 80.73 | | 97.44 |
| | 3 | | | | 80.52 | | 97.26 |
| | 4 | | | | 79.23 | | 101.8 |
| | 5 | | | | 77.34 | | 93.3 |
| | 6 | | | | 76.90 | | 99.5 |
| | 7 | | | | 75.12 | | 92.7 |
| | 8 | | | | 75.46 | | 93.02 |
| | 9 | | | | 82.48 | | 96 |
| | 10 | | | | 82.48 | | 97.4 |
| | 11 | | | | 82.48 | | 96.5 |
| | 12 | | | | 75.00 | | 97.82 |
| | 13 | | | | 74.16 | | 98.24 |
| | 14 | | | | 72.64 | | 92.8 |
| | 15 | | | | 76.80 | | 98.38 |
| | 16 | | | | 71.50 | | 91.57 |

### Analysis of experimental results:

In addition, for the above experiments, this invention also analyzes the data of Preparation Examples 1, 9-11, as shown in Figures 1-3, wherein:
The results in Figure 1 showed that the tear secretion of mice in the DED group (DS5) decreased significantly after DED induction, the tear secretion of the three treatment groups (DS5+AG) was significantly higher than that of the Vehicle control group (DS5+Vehicle), and the DED treatment group (DS5+1%AG) treated with 1% of L-Alanyl-L-Glutamine had the most significant effect, ^{∗∗∗}P<0.001.

The results in Figure 2 showed that the number of conjunctival goblet cells in the mice of DED group (DS5) decreased after the DED induction, and the effect was most significant in the 1% L-Alanyl-L-Glutamine group (preparation case 1), ^{∗}P<0.05_{∘}

The results of Figure 3 showed that there was evident OGD staining in the cornea of the dry eye group (DS5) after modeling, and the OGD staining of the three treatment groups (DS5+AG) were significantly less visible than that of the Vehicle control group (DS5+Vehicle), and the effect was most significant in the 1% L-Alanyl-L-Glutamine group (preparation case 1), * P<0.001.

It should be understood that the invention described herein is not limited to specific methodologies, protocols, or reagents, as these are changeable. The discussion and examples provided herein are presented to illustrate specific embodiments and are not intended to limit the scope of the invention, which is limited only by the claims.

### EMBODIMENTS

Although the present invention is defined in the claims, it should be understood that the present invention can also (alternatively) be defined in accordance with the following embodiments:
1. An ophthalmic pharmaceutical composition comprising of L-Alanyl-L-Glutamine suspended or dissolved in an isoosmotic solution that is suitable for eyes.
2. The pharmaceutical composition of embodiment 1, wherein the concentration of L-Alanyl-L-Glutamine is in the range of 0.1-10% (w/v), preferably in the range of 1-10% (w/v), more preferably in the range of 1- 5% (w/v), most preferably 1% (w/v).
3. The pharmaceutical composition according to embodiment 1 or 2, wherein the isoosmotic solution is prepared with an osmotic agent;
   Preferably, the osmotic agent is selected from one or more of the following: sodium chloride, potassium chloride, boric acid, borax, sodium sulfate, potassium sulfate, sodium nitrate, potassium nitrate, sodium acetate, mannitol, glycerin, propylene glycol, 2-(4-octylphenylethyl)-2-amino-propylene glycol hydrochloride and glucose;
   Preferably, the osmotic agent is selected from either or both sodium chloride and potassium chloride;
   Preferably, the osmotic agent is sodium chloride;
   Preferably, the concentration of the osmotic agent is 0.01-3% (w/v), preferably 0.1-1% (w/v), more preferably 0.4-0.8% (w/v), and most preferably 0.5 %(w/v).
4. The pharmaceutical composition according to any one of embodiments 1 to 3, wherein the pharmaceutical composition optionally contains a bacteriostatic or antimicrobial agent;
   Preferably, the bacteriostatic or antimicrobial agent is selected from one or more of the following: benzalkonium chloride, benzalkonium bromide, chlorhexidine acetate, chlorhexidine gluconate, chlorobutanol, phenoxyethyl alcohol, methyl hydroxybenzoate, ethyl hydroxybenzoate, propyl hydroxybenzoate;
   Preferably, the bacteriostatic or antimicrobial agent is selected from one or more of the following: benzalkonium chloride, benzalkonium bromide and ethyl hydroxybenzoate.
   Preferably, the bacteriostatic or antimicrobial agent is ethyl hydroxybenzoate;
   Preferably, the concentration of the bacteriostatic or antimicrobial agent is 0.003 - 0.5% (w/v), preferably 0.01 - 0.05% (w/v), more preferably 0.02 - 0.035% (w/v), most preferably 0.03 %(w/v).
5. The pharmaceutical composition according to any one of embodiments 1 to 4, wherein the pharmaceutical composition optionally includes a viscosity modifying agent;
   Preferably, the viscosity modifying agent is selected from one or more of the following: sodium hyaluronate, sodium carboxymethyl cellulose, methyl cellulose, polyethylene glycol, polyvinyl alcohol and povidone;
   Preferably, the viscosity increasing agent selected is either or both sodium hyaluronate and sodium carboxymethyl cellulose;
   Preferably, the viscosity modifying agent is sodium hyaluronate.
   Preferably, the concentration of the viscosity modifying agent is 0.01-0.5% (w/v), preferably 0.05-0.2% (w/v), more preferably 0.1-0.15% (w/v), most preferably 0.1 %(w/v).
6. The pharmaceutical composition according to any one of embodiments 1 to 5, wherein the pharmaceutical composition comprises of one or more pH regulators selected from the following: sodium dihydrogen phosphate, disodium hydrogen phosphate, potassium dihydrogen phosphate, dipotassium hydrogen phosphate, boric acid, borax, acetic acid, sodium acetate, citric acid, sodium citrate, tartaric acid, sodium tartrate, sodium carbonate, potassium carbonate, sodium bicarbonate, potassium bicarbonate, sodium hydroxide, potassium hydroxide, hydrochloric acid and phosphoric acid. The pH regulator adjusts the pH value to 5.0-9.0, preferably to 6.0-8.0, more preferably to 6.5-7.5, and most preferably to 7.0;
   Preferably, the pH adjusting agent is selected from one or more of the following: sodium carbonate, sodium bicarbonate and sodium hydroxide.
   Preferably, the pH adjusting agent is sodium hydroxide;
   Preferably, the concentration of sodium hydroxide is 0.25 mol/L.
7. A method of preparing the pharmaceutical composition according to any one of embodiments 1 to 6, wherein the method includes: suspending or dissolving L-Alanyl-L-Glutamine in an isosmotic solution; adjusting the pH to 5.0-9.0, preferably to 6.0 - 8.0, more preferably to 6.5-7.5, most preferably to 7.0; and filtering to sterilize the resulting solution with a microporous filter membrane;
   Preferably, the method includes: stirring and dissolving the isoosmotic agent in Water for Injection, preferably at 80-90°C, most preferably at 85°C; adding L-Alanyl-L-Glutamine; stirring to dissolve, adding Water for Injection; adding a pH adjuster to adjust the pH value to 5.0-9.0, preferably 6.0-8.0, more preferably 6.5-7.5, most preferably 7.0; boiling to sterilize at 100°C ; filtering to sterilize with a microporous filter membrane; and packaging into sterilized eye drop bottles under aseptic manufacturing environment;
   Preferably, the method includes: sequentially stirring in the isoosmotic agent, bacteriostatic agent to water for injection to dissolve, preferably at 80-90°C, most preferably at 85°C; after dissolving, optionally adding a viscosity adjusting agent while stirring and continuing to stir until dissolved; adding L-Alanyl-L-Glutamine and stirring to dissolve; adding water for injection, adding a pH adjuster to adjust the final pH value to 5.0-9.0, preferably 6.0-8.0, more preferably 6.5-7.5, most preferably to 7.0; heating to sterilize at 100°C; filtering to sterilize with a microporous filter membrane; and packaging into sterilized eye drops bottles under aseptic manufacturing environment;
   Preferably, the microporous filter membrane is a 0.22 µm microporous filter membrane.
8. An application of any of the pharmaceutical compositions according to any of embodiments 1 to 6 in the preparation of ophthalmic formulations for relieving DED symptoms and / or improving DED and / or treating DED.
9. A method of relieving DED symptoms and/or improving DED and/or treating DED in a patient suffering from DED therefrom, the method comprising of administering an ophthalmic formulation prepared according to any of embodiments 1 to 6 to said patient.

## Claims

1. An ophthalmic pharmaceutical composition comprising L-Alanyl-L-Glutamine suspended or dissolved in an isoosmotic solution that is suitable for eyes for use in a method of increasing goblet cell density, increasing mucin production, improving corneal epithelial barrier function, reversing corneal damage, and/or increasing tear secretion to treat dry eye disease (DED) and/or ocular surface diseases in a patient in need thereof, the method comprising administering to the patient said pharmaceutical composition.

2. The pharmaceutical composition for use of claim 1, wherein the concentration of L-Alanyl-L-Glutamine is 0.1-10% (w/v) of the composition.

3. The pharmaceutical composition for use of claim 1 or claim 2, wherein the isoosmotic solution comprises an osmotic agent.

4. The pharmaceutical composition for use of claim 3, wherein the osmotic agent is selected from the group consisting of sodium chloride, potassium chloride, boric acid, borax, sodium sulfate, potassium sulfate, sodium nitrate, potassium nitrate, sodium acetate, mannitol, glycerin, propylene glycol, 2-(4-octylphenylethyl)-2-amino-propylene glycol hydrochloride, glucose, and combinations thereof.

5. The pharmaceutical composition for use of claim 3 or claim 4, wherein the concentration of the osmotic agent is 0.01-3% (w/v) of the composition.

6. The pharmaceutical composition for use of any one of claims 1-5, wherein the pharmaceutical composition further comprises a bacteriostatic or antimicrobial agent.

7. The pharmaceutical composition for use of claim 6, wherein the bacteriostatic or antimicrobial agent is selected from the group consisting of benzalkonium chloride, benzalkonium bromide, chlorhexidine acetate, chlorhexidine gluconate, chlorobutanol, phenoxyethyl alcohol, methyl hydroxybenzoate, ethyl hydroxybenzoate, propyl hydroxybenzoate, and combinations thereof.

8. The pharmaceutical composition for use of claim 6 or claim 7, wherein the concentration of the bacteriostatic or antimicrobial agent is 0.003 - 0.5% (w/v) of the composition.

9. The pharmaceutical composition for use of any one of claims 1-8, wherein the pharmaceutical composition further comprises a viscosity modifying agent.

10. The pharmaceutical composition for use of claim 9, wherein the viscosity modifying agent is selected from the group consisting of sodium hyaluronate, sodium carboxymethyl cellulose, methyl cellulose, polyethylene glycol, polyvinyl alcohol, povidone, and combinations thereof.

11. The pharmaceutical composition for use of claim 9 or claim 10, wherein the concentration of the viscosity modifying agent is 0.01-0.5% (w/v) of the composition.

12. The pharmaceutical composition for use of any one of claims 1-11, wherein the pharmaceutical composition further comprises one or more pH regulators.

13. The pharmaceutical composition for use of claim 12, wherein the pH regulator is selected from the group consisting of sodium dihydrogen phosphate, disodium hydrogen phosphate, potassium dihydrogen phosphate, dipotassium hydrogen phosphate, boric acid, borax, acetic acid, sodium acetate, citric acid, sodium citrate, tartaric acid, sodium tartrate, sodium carbonate, potassium carbonate, sodium bicarbonate, potassium bicarbonate, sodium hydroxide, potassium hydroxide, hydrochloric acid, phosphoric acid, and combinations thereof, wherein optionally the pH of the pharmaceutical composition is 5.0-9.0.

14. A method of preparing the pharmaceutical composition of any one of claims 1-13, the method comprising suspending or dissolving L-Alanyl-L-Glutamine in an isosmotic solution; adjusting the pH to 5.0-9.0; and filtering to sterilize the resulting solution with a microporous filter membrane.
